# FASCICULE DE BREVET EUROPEEN

(11) **EP 1 166 720 B1**
(45) Date de publication et mention de la délivrance du brevet: **31.12.2008**
(21) Numéro de dépôt: 01401546.5
(22) Date de dépôt: 14.06.2001
(51) Int. Cl.: A61B 10/00, A61B 17/43

(54) **Dispositif échogène et/ou radio opaque pour le prélèvement ou le transfert dans les organes génitaux**
Echogene oder röntgendichte Vorrichtung zur Entnahme oder zum Transfer in die Genitalorgane
Echogenic or radiopaque device for sampling or tranferring into the genital organs

(30) Priorité: 14.06.2000 FR 0007576
(43) Date de publication de la demande: 02.01.2002
(73) Titulaire: PRODIMED, 60530 Neuilly-en-Thelle (FR)
(72) Inventeur: Choay, Patrick, 75007 Paris (FR); Bouveret, Patrick, 60530 Neuilly en Thelle (FR)
(74) Mandataire: Grosset-Fournier, Chantal Catherine

(56) Documents cités:
- EP-A- 0 456 342
- FR-A- 2 635 453
- FR-A- 2 715 824
- GB-A- 2 118 840
- US-A- 5 492 130
- US-A- 5 800 389

## Description

La présente invention concerne un dispositif echogène et/ou radio opaque apte à permettre d'effectuer des prélèvements dans les organes génitaux, tels que des prélèvements de liquides physiologiques ou de fragments de la paroi interne d'organes génitaux, ou apte à permettre le transfert de produits, notamment de gamètes ou d'embryons, dans les organes génitaux femelles, notamment dans l'utérus ou les trompes.

Les dispositifs de prélèvement couramment utilisés actuellement, comprennent généralement un tube cylindrique ouvert en ses deux extrémités, d'un diamètre extérieur d'environ 3 millimètres et de diamètre interne d'environ 1,5 à 2,6 millimètres, pour une longueur d'environ 25 centimètres.

Ce tube cylindrique se présente de telle façon :
- qu'il peut être relié par le biais d'un raccord (tel qu'un cône Lüer) à une seringue, ou à tout autre dispositif permettant de créer une dépression,
- ou qu'à l'intérieur de ce tube, est apte à coulisser un piston fixé à l'extrémité distale d'une tige, tandis que l'autre extrémité (proximale) de la tige est solidaire d'un organe de préhension.

En variante, le tube des dispositifs susmentionnés peut être ouvert à une première extrémité (extrémité proximale) et comporter à son extrémité opposée (extrémité distale) un ou plusieurs orifices de formes variées, d'un diamètre d'environ 2 millimètres le plus souvent, dit trou d'aspiration ou orifice distal, et prévu sur la paroi cylindrique du tube, c'est-à-dire dans un plan parallèle à l'axe longitudinal du tube. A l'intérieur de ce dernier, est apte à coulisser un piston fixé à l'extrémité distale d'une tige, tandis que l'autre extrémité (proximale) de la tige est solidaire d'un organe de préhension.

Des dispositifs susmentionnés particulièrement avantageux sont caractérisés en ce que sont prévus, du côté de l'extrémité distale du tube, et à proximité du trou d'aspiration, des moyens permettant d'améliorer et augmenter l'action mécanique de prélèvement du tube sur ladite paroi.

L'utilisation, en général à usage unique, de ces dispositifs de prélèvement connus est la suivante :
- dans le cas de prélèvements de liquides physiologiques, notamment dans le cas d'aspirations exo ou endo-cervicales directes, ou pour effectuer par exemple un test de Hühner, après pénétration du col utérin, l'extrémité du dispositif défini ci-dessus est introduite dans la zone de prélèvement : exocol ou endocol, et l'aspiration est réalisée en tirant sur le piston, ou en réalisant le vide par tout moyen approprié; une fois le prélèvement effectué, le dispositif est retiré et le prélèvement restitué en repoussant le piston ;
- dans le cas du prélèvement de fragments de muqueuses, notamment utérines, le dispositif tel que défini ci-dessus est introduit à travers le col de la patiente, dans la cavité utérine. Des graduations prévues sur le tube permettent de localiser approximativement par lecture de celles-ci, la position de l'extrémité distale du tube (pourvu de l'orifice distal). L'opérateur, tout en maintenant le tube en tirant sur la tige, par l'organe de préhension, dans le sens de l'éloignement par rapport à la patiente, réalise une dépression à l'intérieur du tube, et donc un phénomène d'aspiration au niveau de l'orifice disposé à l'extrémité distale du tube. Le prélèvement de fragments de la paroi utérine et de la muqueuse utérine est réalisé en déplaçant le tube, de préférence par mouvement de va-et-vient longitudinal, et de rotation autour de l'axe longitudinal, tout en maintenant l'extrémité distale du tube contre la paroi. Des fragments de muqueuses se trouvent donc arrachés de la paroi et sont aspirés dans le tube au travers de l'orifice distal ou trou d'aspiration. Ce dernier, en vue de côté, dans un plan transversal à l'axe du trou, présente une concavité tournée vers l'extérieur du tube. En d'autres termes, toujours en vue de côté, les bords du trou forment une cuvette dont la concavité est tournée vers l'extérieur du tube.

Une fois l'opération de prélèvement effectuée, l'opérateur retire le dispositif et ensuite déverse le contenu du tube résultant des prélèvements, dans un récipient contenant un liquide pour étude histologique et/ou cytologique.

On comprend que ce type de dispositif doit permettre de prélever des fragments de parois (muqueuses) utérines, de manière fiable, et bien entendu sans douleur. Également, le prélèvement doit être représentatif et donc régulier, en terme de profondeur, dans un plan transversal à la paroi. Le prélèvement doit être également aisé et rapide pour raccourcir au maximum l'opération de prélèvement, compte tenu des désagréments qu'elle comporte pour la patiente.

S'agissant des dispositifs de transfert de gamètes couramment utilisés actuellement, ceux-ci sont des sondes intra-utérines comprenant généralement une sonde en polyéthylène transparent, de longueur d'environ 17 cm terminée par un cathéter très flexible à bout mousse et présentant deux ouvertures latérales opposées; avantageusement un raccord normalisé (tel qu'un cône Lüer) permet la jonction de ce dispositif sur une seringue.

Ce type de dispositif permet d'effectuer une insémination artificielle intra utérine avec sperme préparé. La sonde facilite l'accès dans la cavité utérine sans traumatisme et permet de libérer les spermatozoïdes à proximité des ostiums tubaires. On franchit l'orifice interne du col avec la sonde intra utérine et on injecte le sperme très lentement à préférentiellement 1 cm des ostiums tubaires.

S'agissant des dispositifs de transfert d'embryons couramment utilisés actuellement, ceux-ci comprennent généralement un cathéter en polyéthylène d'une longueur d'environ 17 à 18,5 cm, présentant une extrémité flexible de ⌀ interne 1,1 mm, de ⌀ externe 1,6 mm et de longueur d'environ 4,5 à 5,5 cm, ainsi qu'une ouverture distale.

Pour effectuer le transfert, le cathéter susmentionné est avantageusement relié à une seringue. Les embryons sont chargés dans le cathéter avec un très faible volume de milieu de culture, et le cathéter doit être introduit à 1 cm du fond utérin. Les embryons sont expulsés à l'aide de la seringue.

En variante, des dispositifs de transfert d'embryons comprennent :
- un cathéter d'introduction, de préférence en polypropylène, ayant environ 14,5 cm de long, un diamètre externe d'environ 2,2 mm, présentant des graduations repères à 1, 2, 3, 4, 5, 6 et 7 cm de son extrémité distale, et étant avantageusement muni d'une bague coulissante,
- un cathéter de réimplantation, de préférence en polyuréthanne, ayant environ 23 cm de long, un diamètre externe d'environ 1,53 mm, et un diamètre interne d'environ 0,7 mm, présentant des graduations repères espacées d'un cm à sa partie inférieure, et dont l'embout est avantageusement fermé par un bouchon en polyéthylène translucide.

L'utilisation, en général à usage unique, de ce dispositif de transfert connu est la suivante : on fait pénétrer le cathéter introducteur jusqu'à l'orifice interne du col de l'utérus.

Simultanément, le ou les embryons ont été chargés par le biologiste dans le cathéter de réimplantation.

Ce cathéter de réimplantation est alors introduit dans l'introducteur et sera poussé jusqu'au lieu de transfert.

Lorsque la première marque de couleur déterminée du cathéter de réimplantation affleure l'extrémité proximale du cône Lüer de l'introducteur, leurs deux extrémités distales coïncident. Les marques portées sur l'introducteur à partir de son extrémité distale permettent de calculer la longueur introduite dans l'utérus. Il faudra ajouter le dépassement du cathéter de réimplantation (nombre de cm dont on a dépassé les marques de concordance).

FR 2 715 824 décrit un dispositif pour insémination intra-utérine ou pour transfert ovocytaire comprenant un premier canal ouvert dans sa partie distale au niveau de l'orifice utile pour le prélèvement ou le transfert, et ouvert dans sa partie proximale de manière à pouvoir être relié à un moyen d'aspiration et un deuxième canal fermé dans lequel est logé une tige métallique (8) permettant de raidir partiellement le tube et comportant éventuellement des graduations repères pour contrôler la profondeur de pénétration de la sonde.

FR 2 635 453 décrit un dispositif de cathétérisation des trompes de Fallope par voie basse caractérisé en ce qu'il est constitué par un cathéter de petit diamètre (5), un cathéter d'introduction (2), un mandrin(1) et un dispositif d'injection (7). Ce dispositif évitant les contraintes de la chirurgie est visible sous échographie mais aucune description n'est donnée des moyens de visualisation.

GB 2 118 840 décrit un appareil pour l'intubation du col utérin comprenant un cathéter souple entouré d'un fourreau plus rigide coulissant le log du cathéter. Ce dispositif facilite l'implantation du cathéter au niveau du col de l'utérus.

La présente invention a pour but de fournir des dispositifs de prélèvement ou de transfert tels que définis ci-dessus, et présentant l'avantage, par rapport aux dispositifs existants décrits ci-dessus, de posséder une échogénécité et/ou une radio-opacité spécifiquement située au niveau de l'orifice utile de ces dispositifs pour le prélèvement ou le transfert, et par conséquent de permettre au praticien de localiser bien plus précisément que ne le permettaient les dispositifs décrits ci-dessus, la zone où se fera ce prélèvement ou ce transfert, et de ne plus travailler en aveugle comme cela est le cas actuellement.

La présente invention a également pour but de fournir des dispositifs de prélèvement ou de transfert tels que définis ci-dessus, et présentant l'avantage, par rapport aux dispositifs existants décrits ci-dessus, d'être tels que le matériau échogène et/ou radio-opaque n'est jamais situé à l'extérieur du cathéter de prélèvement et/ou de transfert afin de ne pas gêner l'introduction de ces cathéters dans les organes en question.

L'invention a encore pour but de fournir des dispositifs de prélèvement ou de transfert tels que définis ci-dessus, et présentant l'avantage, par rapport aux dispositifs existants décrits ci-dessus, d'être tels que le matériau échogène et/ou radio-opaque n'est jamais en contact avec les produits prélevés ou transférés, afin de ne pas altérer leurs caractéristiques physico-chimiques ni leur état physiologique.

L'invention concerne tout dispositif échogène et/ou radio opaque apte à permettre d'effectuer des prélèvements dans les organes génitaux en vue d'analyse, tels que des prélèvements de liquides physiologiques ou de fragments de la paroi interne d'organes génitaux mâles ou femelles, plus particulièrement d'organes génitaux féminins, notamment au niveau du col de l'utérus, de l'utérus ou des trompes, ou apte à permettre le transfert de produits tels que ceux choisis parmi les gamètes, ou les embryons, ou les principes actifs, ou les produits utilisés en radiologie dans les organes génitaux femelles, notamment dans les organes génitaux féminins susmentionnés.

L'invention est décrite dans la revendication 1.

Par orifice utile, on entend dans ce qui précède et ce qui suit le ou les trous qui, dans les dispositifs susmentionnés, sont ceux par lesquels les prélèvements sont aspirés, ou les gamètes, embryons, principes actifs, ou produits radiologiques sont expulsés.

Avantageusement, le matériau échogène et/ou radio opaque utilisé dans les dispositifs susmentionnés, est choisi parmi :
- les substances incorporables dans les matériaux en matière plastique constitutifs du dispositif, notamment les sels de baryum, ou de bismuth, ou la poudre de tungstène,
- les films de polymère échogène et /ou radio opaque, recouvrant tout ou partie des dispositifs susmentionnés, tel qu'un revêtement en polytétrafluoroéthylène (PTFE),
- les métaux, notamment ceux à base d'acier inoxydable, ou d'or ou de cuivre.

Avantageusement, dans le cas d'utilisation de métaux, ceux-ci peuvent être recouverts d'un film de polymère échogène, notamment de PTFE, ou voir leur état de surface modifié par tout technique appropriée (par exemple par dépolissage).

L'invention concerne plus particulièrement tout dispositif tel que défini ci-dessus. Un dispositif pour le prélèvement dans les organes génitaux qui ne fait pas partie de la présente invention, est décrit ici. Il correspond à un cathéter de prélèvement comprenant :
* un canal correspondant à un tube cylindrique dont l'extrémité distale (la plus éloignée du manipulateur) est :
   . soit ouverte et constitue l'orifice utile pour le prélèvement de liquides physiologiques,
   . soit obturée à l'exception d'au moins un trou dit d'aspiration, le cas échéant situé en position latérale de l'extrémité distale dudit cathéter, et constituant l'orifice utile pour le prélèvement de fragments de parois d'organes génitaux,
* un piston étanche apte à se déplacer dans ledit tube cylindrique, et relié à l'extrémité distale d'une tige dont l'extrémité proximale est avantageusement pourvue d'un organe de préhension, l'extrémité distale de la tige comportant le piston, ou le piston lui-même, comprenant un matériau échogène et/ou radio opaque, ledit matériau étant situé à la hauteur dudit orifice utile au moment de l'introduction du dispositif (piston poussé et maintenu à la hauteur dudit orifice) dans le col de l'utérus et/ou la cavité utérine.

Avantageusement le matériau échogène et/ou radio opaque utilisé dans le dispositif de prélèvement décrit ci-dessus, est une bague sertie, collée, ou bloquée par surmoulage, ou tout autre moyen, avant, et/ou après, et/ou dans le piston susmentionné.

L'invention a également pour objet tout dispositif de prélèvement ou de transfert tel que défini ci-dessus, ledit dispositif correspondant à un cathéter comprenant :
* un premier canal correspondant à un tube cylindrique dont l'extrémité proximale est susceptible de pouvoir être reliée à une seringue permettant l'aspiration des prélèvements, ou le transfert des produits qu'elle contient, l'extrémité distale de ce canal étant telle qu'elle comprend deux orifices latéraux opposés à titre d'orifice utile pour le transfert, ou étant ouverte et constitue l'orifice utile pour le transfert ou le prélèvement,
* un deuxième canal correspondant à un tube cylindrique dont les extrémités proximale et distale sont fermées, et dans lequel est logé un matériau échogène et/ou radio opaque à la hauteur dudit orifice utile.

L'invention a encore pour objet tout dispositif tel que défini ci-dessus, pour la mise en oeuvre de méthodes d'essais d'introduction de cathéters dans l'utérus, et, le cas échéant, d'analyse de la morphologie du col et de la cavité utérine, ledit dispositif correspondant à un cathéter comprenant :
* un premier canal correspondant à un tube cylindrique dont l'extrémité proximale est ouverte ou fermée, et dont l'extrémité distale est obturée,
* un deuxième canal correspondant à un tube cylindrique dont les extrémités proximale et distale sont fermées, et dans lequel est logé un matériau échogène et/ou radio opaque à la hauteur de l'extrémité distale.

Avantageusement, les diamètres du premier canal et du deuxième canal susmentionnés dans le cadre de dispositifs de prélèvement, de transfert, ou d'essais définis ci-dessus dans le cadre de la présente invention, sont respectivement d'environ 0,9 à environ 1,30 mm, et d'environ 0,4 à environ 0,6 mm.

La longueur des cathéters de prélèvement, de transfert, ou d'essais susmentionnés est de préférence comprise entre environ 150 à environ 300 mm.

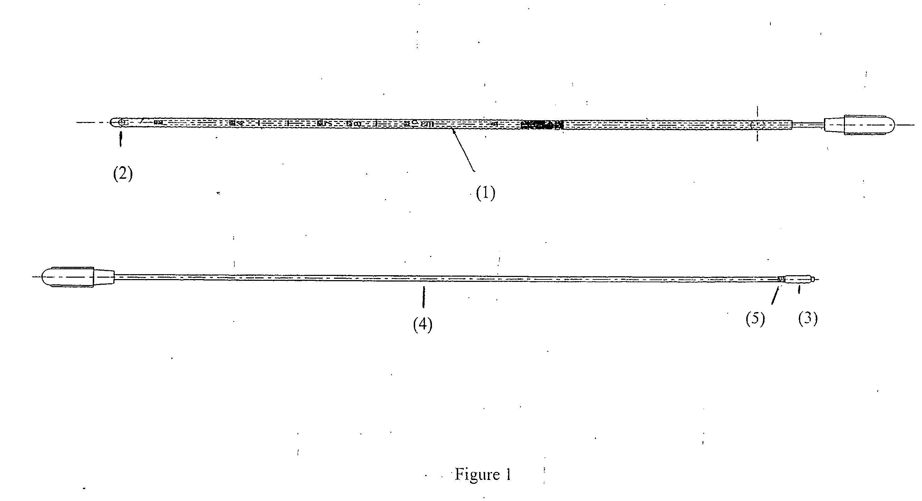

L'invention sera davantage détaillée à l'aide des figures 1 à 3 suivantes :
- figure 1: représentation schématique d'un dispositif de prélèvement; le cathéter de prélèvement est représenté en (1), l'orifice utile en (2) est ici présenté en position latérale, le piston en (3), la tige en (4), et le matériau échogène en (5).

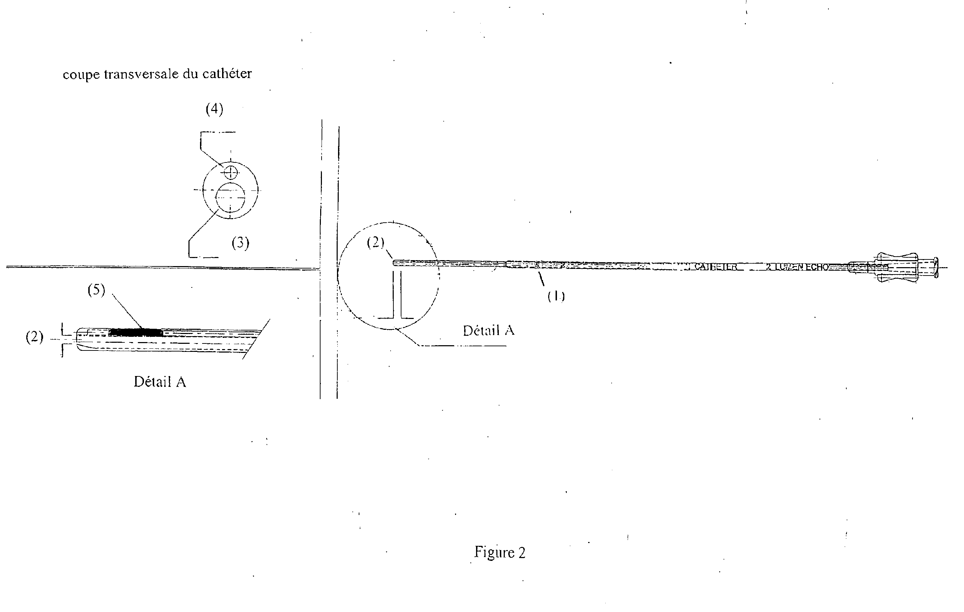

- figure 2 : représentation schématique d'un dispositif de prélèvement ou de transfert selon l'invention ; le cathéter de prélèvement ou de transfert est représenté en (1), l'orifice utile en (2), le premier canal pour le prélèvement ou le transfert en (3), le deuxième canal en (4) contenant le matériau échogène (5) ; lorsque l'orifice utile (2) est obturé, on obtient un dispositif d'essai pour l'étude de la morphologie du col de l'utérus ou de la cavité utérine.

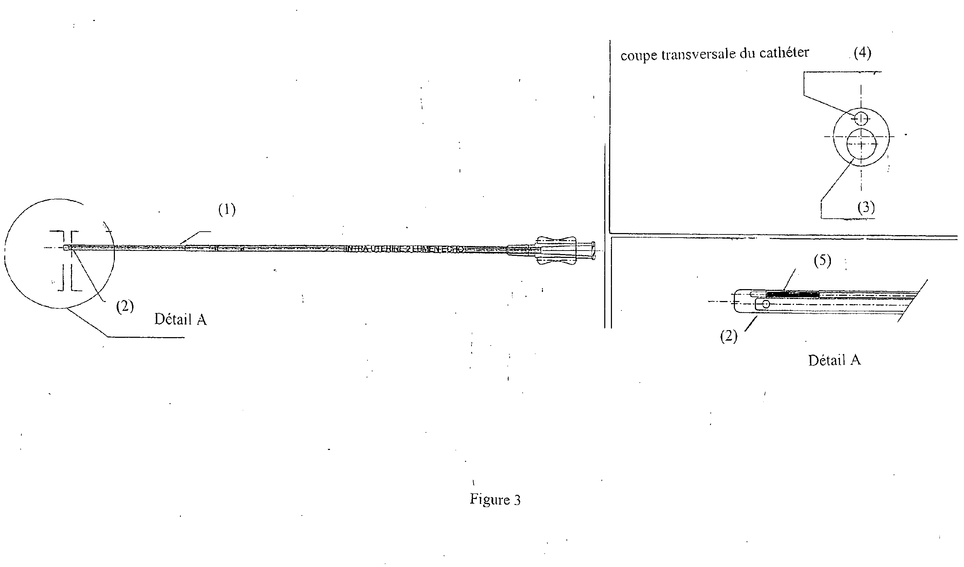

- figure 3 : représentation schématique d'un dispositif de transfert de produits selon l'invention ; le cathéter de transfert est représenté en (1), l'orifice utile constitué de deux orifices opposés en (2), le premier canal pour l'expulsion des produits en (3), le deuxième canal en (4) contenant le matériau échogène (5).

## Revendications

1. Dispositif échogène et/ou radio opaque apte à permettre d'effectuer des prélèvements dans les organes génitaux en vue d'analyse, ou apte à permettre le transfert de produits dans les organes génitaux femelles, ledit dispositif comprenant un cathéter (1) pour le prélèvement ou le transfert susmentionnés, et comportant deux canaux parallèles :
. un premier canal (3) ouvert dans sa partie distale au niveau de l'orifice utile pour le prélèvement ou le transfert, et ouvert dans sa partie proximale de manière à pouvoir être relié à un moyen d'aspiration ou d'expulsion,
. un deuxième canal (4) fermé dans lequel est logé un matériau échogène et/ou radio opaque (5) permettant de visualiser par imagerie médicale, le positionnement de l'orifice utile dudit dispositif pour le prélèvement ou le transfert dans lesdits organes,
**caractérisé en ce que** ledit matériau échogène et/ou radio opaque est spécifiquement situé au niveau dudit orifice utile pour le prélèvement ou le transfert.

2. Dispositif selon la revendication 1 apte à permettre d'effectuer des prélèvements de liquides physiologiques ou de fragments de la paroi interne d'organes génitaux mâles ou femelles.

3. Dispositif selon la revendication 1 apte à permettre le transfert de produits choisis parmi les gamètes, ou les embryons, ou les principes actifs, ou les produits radiologiques dans les organes génitaux femelles.

4. Dispositif selon l'une des revendications 1 à 3, **caractérisé en ce que** le matériau échogène et/ou radio opaque est choisi parmi :
- les substances incorporables dans les matériaux en matière plastique constitutifs du dispositif,
- les films de polymère échogène et /ou radio opaque, recouvrant tout ou partie des dispositifs susmentionnés,
- les métaux, le cas échéant recouverts d'un film de polymère échogène, ou dont la surface est modifiée par toute technique appropriée.

5. Dispositif selon l'une des revendications 1 à 4, **caractérisé en ce que** le matériau échogène et/ou radio opaque est choisi parmi :
- les sels de baryum, ou de bismuth, ou la poudre de tungstène,
- un revêtement en polytétrafluoroéthylène (PTFE),
- les métaux à base d'acier inoxydable, ou d'or ou de cuivre, le cas échéant recouverts d'un film de PTFE.

## Claims

1. An echogenic and/or radio-opaque device adapted to permit carrying out removals from genital organs for analysis, or adapted to permit the transfer of products, into female genital organs, said device comprising a catheter (1) for the said removal or transfer, comprising two parallel channels:
- a first channel (3) opened into its distal portion at the level of the usable orifice for the removal or transfer, and opened into its proximal portion so as to be able to be connected to a suction or expulsion means,
- a second closed channel (4) in which is disposed an echogenic and/or radio-opaque material (5) permitting visualizing by medical imagery, the positioning of the usable orifice of said device for removal from or transfer into said organs,
wherein said echogenic and/or radio-opaque material is located at the height of said usable orifice for the removal or transfer.

2. The device according to claim 1 adapted to permit carrying out removals of physiological liquids or fragments of the walls of male or female genital organs.

3. The device according to claim 1 adapted to permit the transfer of products selected from gametes or embryos or active principle or radiological products into female genital organs.

4. The device according to any of claims 1 to 3, wherein the echogenic and/or radio-opaque material is selected from:
- substances incorporable into plastic material constituting the device,
- echogenic and/or radio-opaque polymeric films covering all or a portion of the above-mentioned devices,
- metals, optionally covered with an echogenic polymeric film or whose surface is modified by any suitable technique.

5. The device according to any of claims 1 to 4, wherein the echogenic and/or radio-opaque material is selected from:
- barium salts, or bismuth salts, or tungsten powder,
- a cladding of polytetrafluoroethylene (PTFE),
- stainless steel based, or gold based or copper based metals, optionally covered with PTFE film.

## Patentansprüche

1. Echogene und/oder strahlenundurchlässige Vorrichtung, die dazu geeignet ist, die Durchführung einer Entnahme in den Geschlechtsorganen zum Zwecke einer Analyse zu ermöglichen, oder dazu geeignet ist, die Übertragung von Produkten in die weiblichen Geschlechtsorgane zu ermöglichen, wobei die Vorrichtung einen Katheter (1) für die obgenannte Entnahme oder Übertragung umfasst und zwei parallele Kanäle umfasst:
einen ersten Kanal (3), der an seinem distalen Abschnitt auf der Höhe der für die Entnahme oder Übertragung nützlichen Öffnung offen ist und an seinem proximalen Abschnitt offen ist, sodass er mit einem Ansaug- oder Abgabemittel verbunden werden kann,
einen zweiten, geschlossenen Kanal (4), in dem ein echogenes und/oder strahlenundurchlässiges Material (5) untergebracht ist, das die Sichtbarmachung durch medizinische Bildgebung der Positionierung der nützlichen Öffnung der Vorrichtung für die Entnahme oder die Übertragung in den Organen ermöglicht,
**dadurch gekennzeichnet, dass** das echogene und/oder strahlenundurchlässige Material spezifisch auf der Höhe der für die Entnahme oder Übertragung nützlichen Öffnung angeordnet ist.

2. Vorrichtung nach Anspruch 1, die dazu geeignet ist, die Entnahme von physiologischen Flüssigkeiten oder Fragmenten der Innenwand von männlichen oder weiblichen Geschlechtsorganen zu ermöglichen.

3. Vorrichtung nach Anspruch 1, die dazu geeignet ist, die Übertragung von Produkten, die aus Gameten, Embryonen, Wirkstoffen oder radiologischen Produkten ausgewählt sind, in die weiblichen Geschlechtsorgane zu ermöglichen.

4. Vorrichtung nach einem der Ansprüche 1 bis 3, **dadurch gekennzeichnet, dass** das echogene und/oder strahlenundurchlässige Material ausgewählt ist aus:
- Substanzen, die in die Kunststoffmaterialien, aus denen die Vorrichtung gebildet ist, eingebaut werden können,
- Filmen aus einem echogenen oder strahlenundurchlässigen Polymer, mit denen die obgenannten Vorrichtungen zur Gänze oder teilweise überzogen sind,
- Metallen, die gegebenenfalls mit einem Film aus einem echogenen oder strahlenundurchlässigen Polymer überzogen sind, oder deren Oberfläche durch ein beliebiges geeignetes Verfahren modifiziert ist.

5. Vorrichtung nach einem der Ansprüche 1 bis 4, **dadurch gekennzeichnet, dass** das echogene und/oder strahlenundurchlässige Material ausgewählt ist aus:
- Barium- oder Wismutsalzen oder Wolframpulver,
- einem Überzug aus Polytetrafluorethylen (PTFE),
- Metallen auf der Basis von rostfreiem Stahl, Gold oder Kupfer, die gegebenenfalls mit einem PTFE-Film überzogen sind.
